(19) Europäisches Patentamt
European Patent Office
Office européen des brevets

(11) Numéro de publication : **0 582 515 A1**

(12) # DEMANDE DE BREVET EUROPEEN

(21) Numéro de dépôt : **93401995.1**

(51) Int. Cl.$^5$ : **C07F 9/38**, A61K 31/66

(22) Date de dépôt : **03.08.93**

(30) Priorité : **05.08.92 FR 9209718**

(43) Date de publication de la demande :
**09.02.94 Bulletin 94/06**

(84) Etats contractants désignés :
**AT BE CH DE DK ES FR GB GR IE IT LI LU NL PT SE**

(71) Demandeur : **ELF SANOFI**
**32-34, rue Marbeuf**
**F-75008 Paris (FR)**

(72) Inventeur : **Bonnery, Michel**
**282 route de Murles**
**F-34570 Vailhauques (FR)**
Inventeur : **Bouisset, Michel**
**23, avenue du 8 mai 1945**
**F-04200 Sisteron (FR)**
Inventeur : **Sole, Raphael**
**11, rue de l'Horloge**
**F-04200 Sisteron (FR)**

(74) Mandataire : **Le Roux, Martine et al**
**Cabinet Beau de Loménie 158, rue de l'Université**
**F-75340 Paris Cédex 07 (FR)**

(54) **Monohydrate du sel disodique de l'acide 4-chlorophénylthiométhylène bisphosphonique, sa préparation, les compositions pharmaceutiques en contenant.**

(57) La présente invention a pour objet le monohydrate du sel disodique de l'acide 4-chlorophénylthiométhylène bisphosphorique.

L'invention concerne également le mode de préparation de ce monohydrate.

L'invention concerne aussi les compositions pharmaceutiques contenant, à titre de principe actif, le monohydrate du sel disodique de l'acide tiludronique.

EP 0 582 515 A1

La présente invention a pour objet le monohydrate du sel disodique de l'acide 4-chlorophénylthiométhylènebisphosphonique.

L'invention concerne également le mode de préparation de ce monohydrate.

Il est connu dans la littérature que certains acides bisphosphoniques et leurs sels peuvent former des hydrates.

Ainsi des hydrates de l'acide hydroxy-1-éthylidènebisphosphonique (Dénomination Commune Internationale : acide étidronique) et de ses sels de sodium sont décrits dans Zh. Obshch. Khim., 1987, 57 (3), 538-544.

La forme cristalline du monohydrate de l'acide diméthylaminométhylènebisphosphonique est décrite dans Kristallografiya, 1990, 35 (6), 1442-9.

Le sel monosodique de l'acide 4-amino-1-hydroxybutylidènebisphosphonique est décrit sous forme de trihydrate dans J. Pharm. Biochem. Anal., 1989, 7 (12), 1719-1727.

Le brevet américain US 4 711 880 et le brevet européen EP 177 443 décrivent la forme cristalline pentahydrate du sel disodique de l'acide 3-amino-1-hydropropylidène-bisphosphonique (Dénomination Commune Internationale : Acide pamidronique) et son mode d'obtention.

Le brevet européen 100 718 décrit des dérivés de l'acide méthylènebisphosphonique de formule :

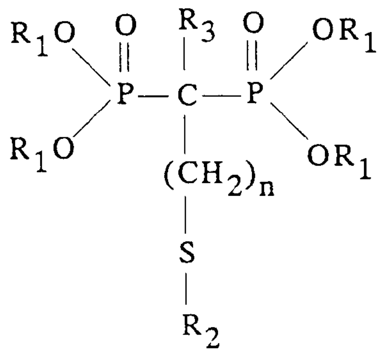

(I)

dans laquelle :

- $R_1$ représente l'hydrogène ou un groupe alkyle inférieur droit ou ramifié ayant de 1 à 4 atomes de carbone ;
- $R_2$ représente :
  . l'hydrogène,
  . un groupe alkyle éventuellement substitué par un groupe hydroxyle, thiol, un ou plusieurs atomes d'halogène, un groupe alcoxycarbonyle ou un groupe

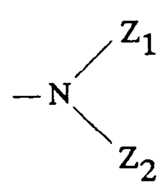

  où $Z_1$ et $Z_2$ considérés indépendamment représentent l'hydrogène ou un groupe alkyle inférieur,
  . un groupe phényle éventuellement substitué une ou plusieurs fois par un halogène ou un groupe nitro, un groupe alkyle inférieur, alcoxy inférieur, trifluorométhyle ou encore par un groupe $NH_2$ ou un groupe COOH ou COO alkyle,
  . un groupe

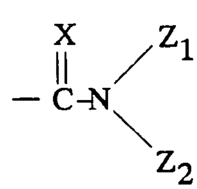

où X désigne l'oxygène ou le soufre, et $Z_1$ et $Z_2$ sont tels que définis ci-dessus,

. un hétérocycle à 5 ou 6 chaînons comportant 1 ou 2 hétéroatomes choisis parmi l'azote et le soufre,

. un hétérocycle à 5 chaînons condensé avec un noyau benzénique et répondant à la formule :

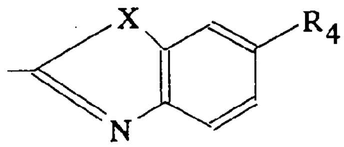

où X peut représenter l'oxygène, un groupe NH ou le soufre et $R_4$ désigne l'hydrogène ou un atome d'halogène de préférence le chlore ;

- $R_3$ désigne l'hydrogène ;
- n représente un nombre entier compris entre O et 10 à condition que :

. $R_2$ soit différent des groupes méthyle ou pyridyle lorsque $R_1$ =H et n = 0,

. $R_2$ soit différent du groupe de formule :

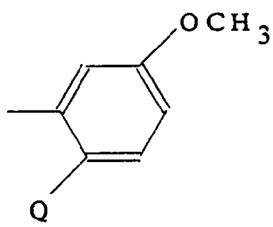

dans lequel Q est un atome d'halogène, un groupe nitro ou un groupe trifluorométhyle lorsque $R_1$ est le groupe méthyle ou éthyle, et n = 0,

. $R_2$ soit différent du groupe phényle lorsque $R_1$ est le groupe éthyle, et n = 0,

. $R_2$ n'est pas un groupe alkyle ou phényle non substitués ou un groupe acétoxy lorsque n = 1, ainsi que les sels desdits dérivés dans lesquels $R_1$ = H avec des bases minérales et organiques.

Ce brevet décrit également le procédé de préparation des composés de formule (I).

Ces composés sont des esters lorsque $R_1$ est un alkyle inférieur ou des acides lorsque $R_1$ est l'hydrogène. Selon le mode opératoire décrit, à partir des composés de formule (I) dans lesquels $R_1$ est un alkyle, on passe au composé (I) dans lesquels $R_1$ représente l'hydrogène par hydrolyse acide. On opère par chauffage au reflux de l'ester dans l'acide chlorhydrique dilué. Après isolement par évaporation, l'acide ainsi obtenu peut être transformé de façon connue en l'un de ses sels.

L'exemple 9 du brevet EP 100 718 décrit la préparation de l'acide 4-chlorophénylthiométhylènebisphosphonique et de son disel de tertiobutylamine.

Le brevet européen EP 336 851 décrit des compositions pharmaceutiques pour administration orale à base d'un dérivé d'acide bisphosphonique en particulier l'acide 4-chlorophénylthiomélhylènebisphosphonique ou son sel disodique, contenant de 1,5 % à 6 % en poids de laurylsulfate de sodium.

L'acide 4-chlorophénylthiométhylènebisphosphonique a reçu la dénomination commune "acide tiludronique" et le sel disodique de cet acide tiludronique, encore appelé tiludronate de sodium, a été choisi pour être développé en tant que médicament utile dans le traitement de la polyarthrite rhumatoide, de la maladie de Paget ou de l'ostéoporose.

L'analyse physico-chimique de ce sel préparé selon le procédé décrit dans EP 100 718 a maintenant montré qu'il s'agit de l'hemihydrate du sel disodique de l'acide tiludronique et non pas du monohydrate comme aurait pu le laisser supposer son analyse élémentaire. Ladite analyse élémentaire, comme indiqué dans Bull. Soc. Chim. Fr., 1988, n°1, pages 49-55, montre en effet que le sel cristallise avec une molécule d'eau. Toutefois, l'analyse physico-chimique montre que ladite molécule d'eau consiste en fait en une demie-molécule d'hydratation et une demie-molécule d'imprégnation prouvant ainsi que le sel de l'art antérieur est un hemihydrate.

De façon tout à fait surprenante, on a maintenant trouvé qu'il existe une nouvelle forme cristalline du sel disodique de l'acide tiludronique. Cette forme cristalline est le monohydrate du sel disodique de l'acide tiludronique et représente l'objet de la présente invention.

L'analyse de cette forme cristalline a montré qu'elle se distingue de la forme précédemment connu du tiludronate de sodium par ses caractéristiques physico-chimiques.

Ainsi, selon un autre objet de la présente invention, le monohydrate du sel disodique de l'acide tiludronique est caractérisé par son spectre IR, par son spectre de rayons X et par sa courbe de perte de masse par thermogravimétrie et son spectre d'ACD qui présente un pic endothermique à 121+/-8°C.

Le procédé d'obtention du monohydrate du sel disodique de l'acide tiludronique est également un object de la présente invention. Ce procédé est caractérisé en ce que :

on chauffe le sel disodique de l'acide tiludronique sous forme d'hémihydrate en solution aqueuse à une température comprise entre 60°C et 90°C, pendant un temps compris entre 2 heures et 24 heures, on laisse refroidir jusqu'à une température comprise entre la température ambiante et 5°C, on filtre le précipité formé puis on le sèche.

Selon un mode de réalisation particulier, le sel disodique de l'acide tiludronique sous forme de monohydrate peut être préparé in situ, à partir d'une solution aqueuse d'acide tiludronique par addition d'une quantité de soude suffisante pour atteindre un pH compris entre 4,6 et 4,8.

Le chauffage du sel disodique de l'acide tiludronique sous forme d'hemihydrate en solution aqueuse peut être effectué en présente d'un solvant miscible à l'eau, choisi parmi : l'acétone, l'éthanol ou l'alcool isopropylique.

La dilution du sel disodique de l'acide tiludronique sous forme d'hémihydrate dans la solution aqueuse peut varier dans un rapport de 1 en poids de sel pour 2 à 10 volumes d'eau.

Lorsque l'on utilise un solvant miscible à l'eau, le volume de celui-ci peut varier dans un rapport de 1 à 25 vis-à-vis du poids du sel disodique de l'acide tiludronique et, indépendamment, dans un rapport de 0,5 à 2,5 vis-à-vis du volume d'eau.

Selon une variante du procédé, on chauffe l'hémihydrate du sel disodique de l'acide tiludronique sous forme cristalline, réparti en couche mince, en présence d'eau, dans une enceinte fermée chauffée entre 65°C et 75°C pendant 1 à 5 jours, puis on sèche.

Le séchage du composé formé peut être effectué dans une étuve ventilée à une température comprise entre 50°C et 70°C. Il peut également être complété par un séchage azéotropique en présence d'un solvant approprié. Comme solvants utiles pour un séchage azéotropique, on peut citer par exemple : l'éther isopropylique, l'acétate d'éthyle, la méthyl-3-butanonc, le n-butanol, le dichloroéthane ou le toluène.

Le monohydrate du sel disodique de l'acide tiludronique est particulièrement stable dans le temps, quelles que soient les conditions d'humidité et de température.

Le monohydrate permet de préparer des compositions pharmaceutiques, objets de la présente invention qui sont également particulièrement stables.

Les compositions pharmaceutiques selon la présente invention comprennent notamment les comprimés pour administration par voie orale.

Les compositions pharmaceutiques selon la présente invention peuvent contenir en outre des ingrédients utilisés habituellement en pharmacie par exemple un agent de désintégration, un agent d'écoulement ou tout excipient de masse convenable.

Comme excipient de masse, on peut utiliser le lactose, la cellulose ou les amidons. Comme lubrifiant on peut utiliser l'acide stéarique, le stéarate de magnésium, la L-leucine ou, par exemple, le tribéhénate de glycérol. Comme agent de désintégration, on peut utiliser le carboxyméthylamidon sodique, la carboxyméthylcellulose sodique réticulée ou, par exemple, la polyvinylpyrrolidone réticulée. Comme agent d'écoulement, on peut utiliser la silice pure ou le dioxyde de silicium colloïdal.

La présente invention se réfère également aux formes orales à dissolution instantanée et aux formes orales effervescentes obtenues en ajoutant un couple effervescent à la composition selon l'invention. Comme couple effervescent, on peut utiliser, par exemple, l'acide tartrique et le bicarbonate de sodium ou l'acide citrique et le bicarbonate de sodium.

La forme comprimé est une forme préférée selon l'invention. L'invention se réfère également aux comprimés à dissolution instantanée, aux comprimés effervescents ainsi qu'aux comprimés recouverts d'un enrobage.

Dans la suite de la description, on convient d'appeler forme 1, l'hémihydrate du sel disodique de l'acide tiludronique, telle que connu précédemment et forme 2, le monohydrate dudit sel disodique de l'acide tiludronique, objet de la présente invention.

Par ailleurs, les abréviations suivantes sont utilisées :

TA : Température ambiante
l: litre
ACD : analyse calorimétrique différentielle.

Les formes 1 et 2 du sel disodique de l'acide tiludronique obtenues ci-après ont un point de fusion supérieur à 250°C.

A) Préparation de l'acide 4-chlorophénylthiométhylènebisphosphonique.

En applicant le procédé décrit dans le brevet EP 100 718, on prépare le 4-chlorophénylthiométhylènebisphosphonate de tétraisopropyle. Ce composé est traité par de l'eau et de l'acide chlorhydrique et chauffé à 90°C pendant plusieurs heures. Après refroidissement, on extrait par du dichloroéthane. On concentre la phase aqueuse sous vide pour éliminer l'acide chlorhydrique et l'eau. On ajoute du toluène et distille pour chasser l'eau résiduelle. L'acide 4-chlorophénylthiométhylènebisphosphonique précipite au refroidissement. On filtre pour recueillir ce précipité, on le lave au toluène et on le sèche à une température inférieure ou égale à 70°C.

B) Préparation du sel disodique de l'acide 4-chlorophénylthiométhylènebisphosphonique (forme 1).

A une suspension de l'acide obtenu précédemment dans de l'eau, on ajoute de la soude soit en pastille, soit en solution aqueuse pour atteindre un pH d'environ 4,7. On ajoute du charbon actif et on clarifie par filtration. Le filtrat est mélangé avec de l'acétone et le sel attendu précipite à TA.

On le recueille par filtration, lave par de l'acétone et sèche à une température inférieure ou égale à 70°C.

A partir de la solution aqueuse à pH 4,7, on peut également faire précipiter le sel par addition d'éthanol puis refroidissement du milieu à 10°C.

Différentes méthodes physicochimiques sont utilisées pour analyser et caractériser le sel ainsi obtenu.

. IR, enregistré à 1 % (m/v) dans le bromure de potassium : figure 1.

| | |
|---|---|
| 1600 $cm^{-1}$ et 1480 $cm^{-1}$ : | C-C aromatiques |
| 1390 $cm^{-1}$ : | C-H |
| 1090 $cm^{-1}$ et 1050 $cm^{-1}$ : | P-O |
| 820 $cm^{-1}$ : | C-H |
| 710 $cm^{-1}$ : | C-S. |

. RMN enregistré à 250 MHz dans le $D_2O$ : figure 2.

. Analyse élémentaire : $C_7H_5ClO_6P_2SNa_2$.

teneur en eau : 4,9 %

| | | | | |
|---|---|---|---|---|
| théorique | C : 23,19 | H : 1,95 | S : 8,84 | Cl : 9,78 |
| mesuré | C : 21,65 | H : 2,26 | S : 8,54 | Cl : 9,18 |
| corrigé | C : 22,76 | H : 1,80 | S : 8,98 | Cl : 9,65 |

La valeur "corrigée" est obtenue à partir de la valeur "mesurée" en prenant en compte la teneur en eau.

La teneur en eau indique la présence de l'équivalent d'une molécule d'eau par molécule de produit, correspondant à une demie-molécule d'hydratation et à une demie-molécule d'imprégnation, comme indiqué ci-après.

. Courbe de perte de masse par thermogravimétrie : figure 3.

La thermogravimétrie est réalisée sur appareil Perkin-Elmer TGA 7, équipé d'une station de traitement de données. L'étalonnage est effectué par rapport aux points de curie de l'alumel et du nickel. La vitesse de chauffage est de 10,0°C par minute et le domaine de température est 40°C à 200°C. L'azote est le gaz de purge de la balance. La prise d'essai est de 5,160 mg.

Sur la figure 3, on constate que la courbe indique deux pertes de masse successives :

- entre 40°C et 90°C, on constate la perte de 0,5 mole d'eau d'imprégnation,
- entre 90°C et 160°C, on constate la perte de 0,5 mole d'eau d'hydratation.

On peut en déduire que la forme 1 est l'hémihydrate du sel disodique de l'acide tiludronique qui comprend environ une demie molécule d'eau supplémentaire qui correspond à la demie-molécule d'imprégnation.

. Analyse calorimétrique différentielle : figure 4.

Cette analyse est effectuée sur un appareil Setaram DSC 101, vendu par Setaram, Lyon, France. La température initiale est de 20°C, elle monte jusqu'à 150°C à une vitesse de 1,0°C par minute. La prise d'essai est de 10 mg.

. Spectre de diffraction des rayons X : figure 5.

Le diagramme de poudre est réalisé sur un diffractomètre Siemens D 500 TT, de type théta-théta, muni d'une anode de cuivre, pour des valeurs de 2 théta comprises entre 4° et 40°.

Le spectre présente une raie principale à 5,4 et des raies moins intenses à 16,3 ; 21,8 et 20,1 comme indiqué dans le tableau 1 ci-après.

TABLEAU 1

| 2theta | d | I rel. | 2theta | d | I rel. |
|---|---|---|---|---|---|
| 5.408 | 16.3281 | 100.0 | 5.408 | 16.3281 | 100.0 |
| 10.841 | 8.1546 | 8.2 | 16.306 | 5.4317 | 13.1 |
| 12.011 | 7.3626 | 4.4 | 21.806 | 4.0725 | 10.4 |
| 16.306 | 5.4317 | 13.1 | 20.110 | 4.4121 | 8.9 |
| 16.877 | 5.2491 | 6.4 | 10.841 | 8.1546 | 8.2 |
| 17.910 | 4.9485 | 7.7 | 17.910 | 4.9485 | 7.7 |
| 20.110 | 4.4121 | 8.9 | 30.067 | 2.9697 | 7.4 |
| 21.806 | 4.0725 | 10.4 | 30.230 | 2.9541 | 7.0 |
| 24.144 | 3.6832 | 6.6 | 24.144 | 3.6832 | 6.6 |
| 27.242 | 3.2710 | 6.5 | 27.242 | 3.2710 | 6.5 |
| 28.760 | 3.1017 | 6.0 | 16.877 | 5.2491 | 6.4 |
| 30.067 | 2.9697 | 7.4 | 32.837 | 2.7253 | 6.0 |
| 30.230 | 2.9541 | 7.0 | 28.760 | 3.1017 | 6.0 |
| 32.837 | 2.7253 | 6.0 | 12.011 | 7.3626 | 4.4 |
| 34.928 | 2.5668 | 4.4 | 34.928 | 2.5668 | 4.4 |

Les exemples suivants décrivent la préparation du monohydrate du sel disodique de l'acide tiludronique (forme 2) par application du procédé selon l'invention.

EXEMPLE 1

On prépare une solution aqueuse du sel disodique de l'acide tiludronique (forme 1) en mélangeant 100 g du sel dans 1 litre d'eau et on filtre cette solution pour la rendre limpide. On porte à reflux 2,3 litres d'acétone et l'on verse dessus la solution aqueuse précédente tout en maintenant le reflux pendant 3 heures. On laisse revenir le milieu à température ambiante puis on le refroidit pendant 1 heure à 10°C. Le précipité formé est filtré puis séché une nuit à 60°C dans une étuve ventilée. On obtient 95 g de produit attendu sous forme cristalline.

. IR enregistré à 1 % (m/v) dans le bromure de potassium : figure 6.

Certaines bandes sont les mêmes que celles observées avec la forme 1, d'autres sont différentes.

| | |
|---|---|
| 1600 $cm^{-1}$ et 1480 $cm^{-1}$ : | C-C aromatiques |
| 1390 $cm^{-1}$ : | C-H |
| 1090 $cm^{-1}$ et 1050 $cm^{-1}$ : | P-O |
| 820$cm^{-1}$ : | C-H |
| 710 $cm^{-1}$ : | C-S. |

nouvelles bandes :
3600 $cm^{-1}$
3400 $cm^{-1}$
2920 $cm^{-1}$
1300 $cm^{-1}$
900 cm-$^{-1}$

. Le spectre RMN (figure 7) est identique à celui enregistré avec la forme 1 dans les mêmes conditions.

. Analyse élémentaire : $C_7H_5ClO_6P_2SNa_2$, $H_2O$

| théorique | C : 22,09 | H : 2,38 | S : 8,42 | Cl : 9,31 | $H_2O$ : 4,72 |
|---|---|---|---|---|---|
| trouvé | C : 21,99 | H : 2,54 | S : 8,40 | Cl : 9,80 | $H_2O$ : 4,77 |

. Courbe de perte de masse par thermogravimétrie : figure 8.

La prise d'essai est de 5,178 mg. Les conditions opératoires sont identiques à celles indiquées pour la forme 1.

La courbe présente une perte de masse entre 90°C et 160°C qui correspond à la perte d'une mole d'eau d'hydratation.

. Analyse calorimétrique différentielle : figure 9.

Les conditions sont celles décrites pour la forme 1.

On observe un pic endothermique centré à 129°C.

. Spectre de diffraction des rayons X : figure 10.

Le diagramme de poudre est obtenu dans les mêmes conditions que pour la forme 1. Le spectre présente une raie principale à 5,6 et des raies moins intenses à; 29,0 ; 22,6 et 16,9, comme indiqué dans le tableau 2 ci-après.

TABLEAU 2

| 2theta | d | I rel. | 2theta | d | I rel. |
|---|---|---|---|---|---|
| 5.604 | 15.7570 | 100.0 | 5.604 | 15.7570 | 100.0 |
| 11.220 | 7.8797 | 9.6 | 29.015 | 3.0750 | 34.2 |
| 14.564 | 6.0772 | 10.2 | 22.572 | 3.9360 | 33.9 |
| 15.415 | 5.7435 | 25.2 | 16.876 | 5.2493 | 31.2 |
| 16.272 | 5.4430 | 14.7 | 19.386 | 4.5750 | 26.4 |
| 16.876 | 5.2493 | 31.2 | 29.274 | 3.0483 | 25.3 |
| 17.124 | 5.1740 | 20.5 | 15.415 | 5.7435 | 25.2 |
| 19.386 | 4.5750 | 26.4 | 31.085 | 2.8748 | 22.6 |
| 20.298 | 4.2413 | 17.3 | 17.124 | 5.1740 | 20.5 |
| 22.572 | 3.9360 | 33.9 | 34.088 | 2.6281 | 19.9 |
| 24.149 | 3.6825 | 17.2 | 20.928 | 4.2413 | 17.3 |
| 25.274 | 3.5209 | 14.5 | 24.149 | 3.6825 | 17.2 |
| 26.108 | 3.4103 | 14.9 | 28.035 | 3.1802 | 16.6 |
| 26.703 | 3.3358 | 14.9 | 28.286 | 3.1526 | 15.8 |
| 28.035 | 3.1802 | 16.6 | 31.733 | 2.8175 | 15.1 |
| 28.286 | 3.1526 | 15.8 | 26.703 | 3.3358 | 14.9 |
| 29.015 | 3.0750 | 34.2 | 26.108 | 3.4103 | 14.9 |
| 29.274 | 3.0483 | 25.3 | 16.272 | 5.4430 | 14.7 |
| 29.939 | 2.9821 | 12.5 | 25.274 | 3.5209 | 14.5 |
| 30.519 | 2.9267 | 7.4 | 33.226 | 2.6942 | 13.8 |
| 31.085 | 2.8748 | 22.6 | 29.939 | 2.9821 | 12.5 |

| | | | | | |
|---|---|---|---|---|---|
| 31.733 | 2.8175 | 15.1 | 34.809 | 2.5753 | 12.3 |
| 33.226 | 2.6942 | 13.8 | 38.661 | 2.3270 | 10.6 |
| 34.088 | 2.6281 | 19.9 | 14.564 | 6.0772 | 10.2 |
| 34.809 | 2.5753 | 12.3 | 11.220 | 7.8797 | 9.6 |
| 36.571 | 2.4551 | 6.8 | 37.278 | 2.4102 | 7.4 |
| 37.278 | 2.4102 | 7.4 | 30.519 | 2.9267 | 7.4 |
| 38.661 | 2.3270 | 10.6 | 39.654 | 2.2711 | 7.1 |
| 39.654 | 2.2711 | 7.1 | 36.571 | 2.4551 | 6.8 |

La comparaison des résultats analytiques obtenus avec les formes 1 et 2 montre qu'il s'agit de 2 formes cristallines différentes. En effet, bien que l'analyse élémentaire montre la présence d'une molécule d'eau dans chacune des formes, seule la forme 2, obtenue par le procédé selon l'invention est un monohydrate.

Les exemples suivants décrivent d'autres modes de préparation du monohydrate de sel disodique de l'acide tiludronique. Le produit selon l'invention obtenu est caractérisé par le pic endothermique observé par l'analyse calorimétrique différentielle (ACD). Pour chacun des produits obtenus, on a vérifié la conformité du spectre IR avec celui de l'exemple 1.

EXEMPLE 2

On met en suspension 100 g d'acide tiludronique dans 0,3 litre d'eau et on chauffe à 60°C. On ajuste le pH du milieu entre 4,6 et 4,8 par ajout d'une solution aqueuse de soude à 10 %. On laisse stabiliser le pH pendant 2 heures puis on filtre la solution obtenue. On porte la solution vers 60°C en ajoutant lentement 0,7 litre d'acétone et on maintient le reflux pendant 3 heures. On laisse le milieu à température ambiante puis on le porte à 10°C pendant 1 heure. Le précipité formé est filtré puis séché en étuve ventilée à 60°C.

On obtient 93 g du sel dans la forme cristalline attendue (forme 2).

ACD : pic endothermique centré à 128°C.

EXEMPLES 3 à 6

On mélange 100 g de sel disodique de l'acide tiludronique (forme 1), avec plusieurs volumes d'eau (nV), on porte à 70°C puis on ajoute plusieurs volumes d'acétone (n'V) et l'on maintient le reflux pendant plusieurs heures. On laisse revenir à température ambiante puis on filtre le précipité formé et on le sèche en étuve ventilée à 60°C.

Les différents exemples sont rassemblés dans le tableau 3 ci-après ; pour chacun d'eux on a indiqué le rendement obtenu en sel disodique de l'acide tiludronique (forme 2).

TABLEAU 3

| Exemple n° | Eau nV (l) | Acétone n'V (l) | V total (l) | Rendement | ACD °C |
|---|---|---|---|---|---|
| 3 | 0,3 | 0,2 | 0,5 | 93g | 129 |
| 4 | 0,4 | 0,4 | 0,8 | 87 g | 122 |
| 5 | 0,4 | 0,7 | 1,1 | 85 g | 122 |
| 6 | 0,3 | 0,7 | 1,0 | 85 g | 120 |

EXEMPLES 7 et 8

On mélange sous agitation 100 g de sel disodique de l'acide tiludronique (forme 1) et 0,4 litre d'eau et l'on chauffe à 90°C. Après 1 heure, on filtre pour rendre la solution limpide puis on verse dans la solution 0,7 litre

d'acétone en laissant la température s'abaisser vers 60°C. Après 1 heure à 60°C, on laisse revenir à température ambiante puis on essore le précipité présent; le précipité humide ainsi obtenu est repris par 1 litre d'un solvant permettant un séchage azéotropique sous pression atmosphérique.

Les solvants utilisés sont soit l'acétate d'éthyle soit l'éther isopropylique qui permettent d'obtenir le sel dans la forme cristalline attendue (forme 2) avec les rendements suivants: 89 % et 97 %, ACD : pic endothermique centré à 118°C.

EXEMPLES 9 à 13

On porte 0,2 litre d'eau à 80°C et on y ajoute en 1 heure 100 g de sel disodique d'acide tiludronique (forme 1). Après 5 heures à 80°C, on abaisse la température de 5°C par heure jusqu'à 20°C et on filtre le précipité formé. Ce précipité est séparé en plusieurs parties qui sont séchées par différents moyens pour obtenir le sel dans la forme cristalline attendue (forme 2).

- Séchage à 60°C dans une étuve ventilée, rendement : 75 %
- Séchage azétropique
  avec le méthyl-3-butanone, rendement : 91 %
  avec le n-butanol, rendement : 89 %
  avec le dichloroéthane, rendement : 93 %, ACD : pic endothermique centré à 113°C
  avec le toluène, rendement : 97 %, ACD : pic endothermique centré à 117°C.

EXEMPLE 14

100 g du sel disodique de l'acide tiludronique (forme 1) sont broyés, la poudre est étalée dans un cristallisoir de 23 cm de diamètre sur une hauteur de 0,5 cm. Le cristallisoir est placé dans un dessicateur dont la partie basse contient de l'eau. Le dessicateur est fermé et placé pendant 3 jours dans une étuve à 65-70°C. La poudre contenue dans le cristallisoir est alors séchée pendant une journée dans une étuve ventilée à 50°C.

On obtient 100 g du sel dans la forme cristalline attendue (forme 2). La structure est confirmée par les résultats analytiques : les spectres IR, ATG et diffraction RX sont conformes à ceux obtenus à l'exemple 1.

EXEMPLE 15

La stabilité dans le temps du monohydrate du sel disodique de l'acide tiludronique a été étudiée en fonction des conditions variables de température et d'humidité.

Aucun produit de dégradation n'est apparu après 6 mois de conservation dans des conditions normales de température et d'humidité, ce qui montre la bonne stabilité chimique du monohydrate. On a étudié la stabilité physique de cette forme cristalline dans les conditions explicitées ci-après.

Dans le fond d'un vase fermant hermétiquement, on place une solution saline saturée permettant d'obtenir l'humidité relative désirée à différentes températures. Sur un support placé au dessus de la solution saline saturée, on dispose 500 mg de la forme 2 à étudier. Après plusieurs jours (j) à une température déterminée (T°C) et dans une humidité relative déterminée (HR), on mesure l'augmentation de la masse (ΔM %) et on vérifie par analyse Karl-Fischer que l'augmentation de la masse est due à l'augmentation de la teneur en eau.

Les résultats obtenus sont reportés dans le tableau 4 ci-après :

TABLEAU 4 (Forme 2)

| HR % / T°C,temps | 33 | 43 | 53 | 58 | 75 | 85 | |
|---|---|---|---|---|---|---|---|
| 50°C, 49 jours | 0<br>(2) | 0<br>(2) | 0<br>(2) | 0<br>(2) | 0<br>(2) | 0,3<br>(2) | ΔM %<br>Identification |
| 35°C, 41 jours | 0<br>(2) | 0<br>(2) | 0<br>(2) | 0<br>(2) | 0<br>(2) | 0<br>(2) | ΔM %<br>Identification |
| 25°C, 58 jours | 0<br>(2) | 0<br>(2) | 0,5<br>(2) | 0,55<br>(2) | 0,9<br>2) | 1,4<br>(2) | ΔM %<br>Identification |

Les résultats montrent que la forme 2, c'est-à-dire le monohydrate du sel disodique de l'acide tiludronique est stable. La seule variation observée est une légère augmentation de la masse (environ 1 %) à 25°C, dans une humidité relative supérieure à 75 % et après 58 jours.

EXEMPLE 16

On prépare en granulation sèche un comprimé ayant la composition suivante :

| | |
|---|---|
| monohydrate de sel disodique de l'acide tiludronique : | 240 mg |
| lauryl sulfate de sodium : | 4,5 mg |
| silice colloïdale anhydre : | 1,3 mg |
| cellulose microcristalline : | 21,6 mg |
| polyvinylpyrrolidone réticulée : | 8,0 mg |
| stéarate de magnésium : | 5,6 mg |
| lactose anhydre pour un comprimé à : | 320 mg |

EXEMPLE 17

On prépare en granulation humide un comprimé ayant la composition suivante :

| | |
|---|---|
| monohydrate du sel disodique de l'acide tiludronique : | 240 mg |
| lauryl sulfate de sodium : | 4,5 mg |
| méthylhydroxypropylcellulose : | 5,25 mg |
| polyvinylpyrrolidone réticulée : | 15,0 mg |
| stéarate de magnésium : | 0,60 mg |
| eau purifié | |
| lactose | |
| pour un comprimé terminé à : | 300 mg |

Les comprimés des exemples 17 et 18 ont été conservés pendant 15 mois à température ambiante. Après ce temps de conservation, les résultats des études analytiques effectuées sur la poudre de comprimés indiquent que le sel disodique de l'acide tiludronique (forme 2) est stable dans la forme galénique.

Les spectres IR et ATG des comprimés des exemples 17 et 18 sont conformes à ceux obtenus à l'exemple 1.

## Revendications

**1.** Monohydrate du sel disodique de l'acide tiludronique.

**2.** Monohydrate du sel disodique de l'acide tiludronique caractérisé par :
- son spectre IR enregistré à 1 % (m/v) dans le bromure de potassium et comprenant les bandes suivantes : 3600, 3400, 2920, 1600, 1480, 1390, 1300, 1090, 1050, 900, 820, 710 $cm^{-1}$ ;
- son spectre de diffraction des rayons X mesuré sur la poudre cristalline et comprenant une raie principale pour une valeur de 2θ de 5,60 et des raies à 29,0 ; 22,6 et 16,9 ;
- son spectre d'ACD qui présente un pic endothermique à 121 +/- 8°C ;
- sa courbe de perte de masse par thermogravimétrie représentée à la figure 8.

**3.** Procédé pour la préparation du monohydrate du sel disodique de l'acide tiludronique selon l'une des revendications 1 ou 2 caractérisé en ce que
on chauffe le sel disodique de l'acide tiludronique sous forme d'hemihydrate en solution aqueuse à une température comprise entre 60° et 90°C, pendant un temps compris entre 2 heures et 24 heures, on laisse refroidir jusqu'à une température comprise entre la température ambiante et 5°C, on filtre le précipité formé, puis on le sèche.

**4.** Procédé selon la revendication 3 caractérisé en ce que le sel disodique de l'acide tiludronique sous forme d'hemihydrate est préparé in situ à partir d'une solution aqueuse d'acide tiludronique par addition d'une quantité de soude suffisante pour atteindre un pH compris entre 4,6 et 4,8.

**5.** Procédé selon l'une des revendications 3 ou 4 caractérisé en ce que le chauffage est effectué en présence d'un solvant choisi parmi l'acétone, l'éthanol ou l'alcool isopropylique.

**6.** Procédé selon l'une des revendications 3 à 5 caractérisé en ce que le sel disodique de l'acide tiludronique sous forme d'hemihydrate utilisé est dilué dans la solution aqueuse dans un rapport de 1 en poids pour 2 à 10 en volumes.

**7.** Procédé selon l'une quelconque des revendications 3 à 6 caractérisé en ce que le volume de solvant est utilisé dans un rapport pouvant varier de 1 à 25 vis-à-vis du poids du sel disodique de l'acide tiludronique et, indépendamment, dans un rapport pouvant varier de 0,5 à 2,5 vis-à-vis du volume d'eau.

**8.** Procédé pour la préparation du monohydrate du sel disodique de l'acide tiludronique selon l'une des revendications 1 ou 2 caractérisé en ce que l'on chauffe l'hemihydrate du sel disodique de l'acide tiludronique sous forme cristalline, réparti en couche mince, en présence d'eau, dans une enceinte fermée chauffée entre 65°C et 75°C pendant 1 à 5 jours, puis on sèche.

**9.** Procédé selon l'une des revendications 3 à 8 caractérisé en ce que l'on effectue à la dernière étape, soit un séchage azéotropique en présence d'un solvant, soit un séchage par ventilation à une température comprise entre 50°C et 70°C.

**10.** Compositions pharmaceutiques contenant à titre de principe actif le monohydrate du sel disodique de l'acide tiludronique selon l'une des revendications 1 ou 2.

Fig. 1

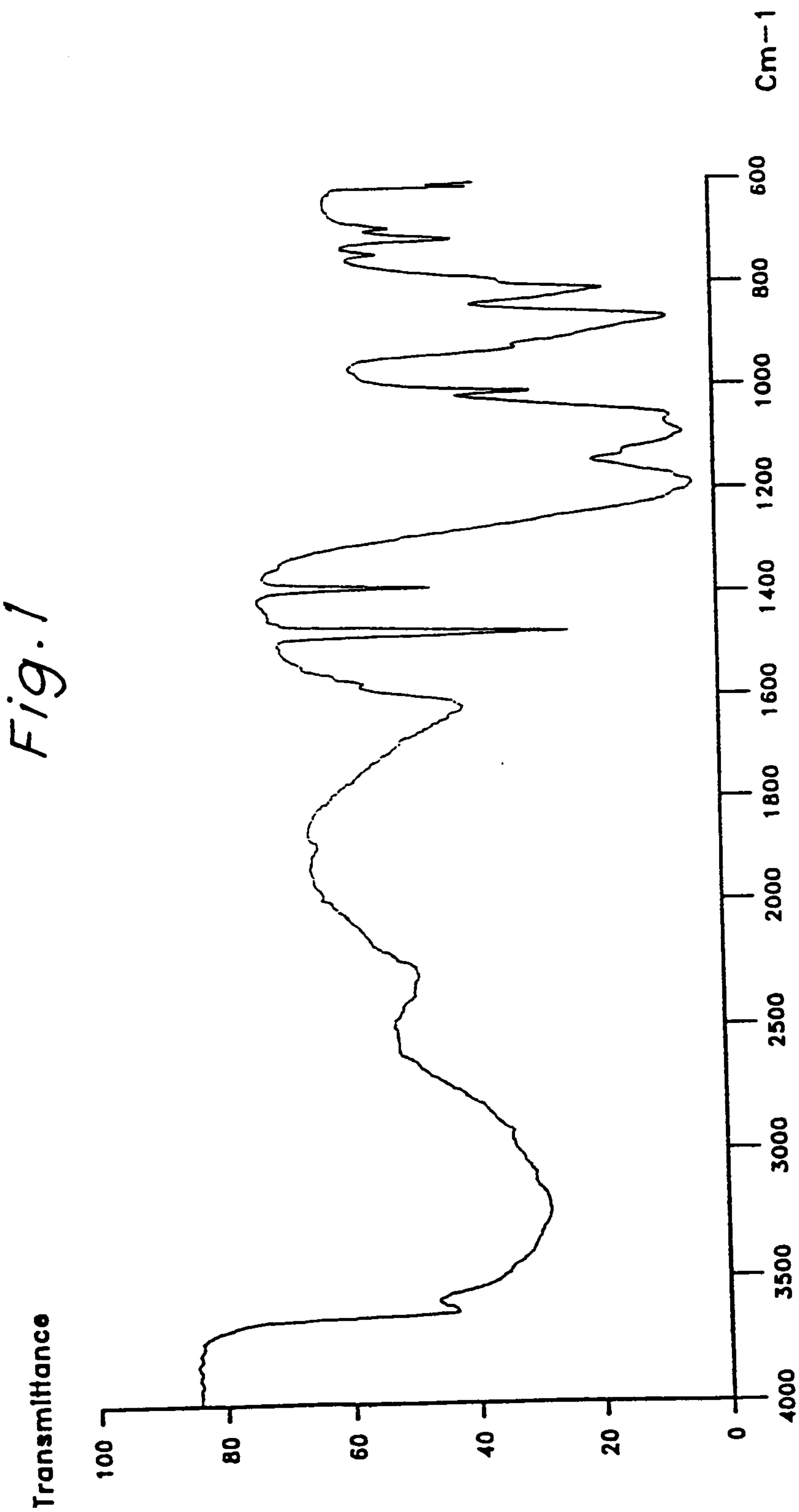
Transmittance
100
80
60
40
20
0
4000
3500
3000
2500
2000
1800
1600
1400
1200
1000
800
600
Cm-1

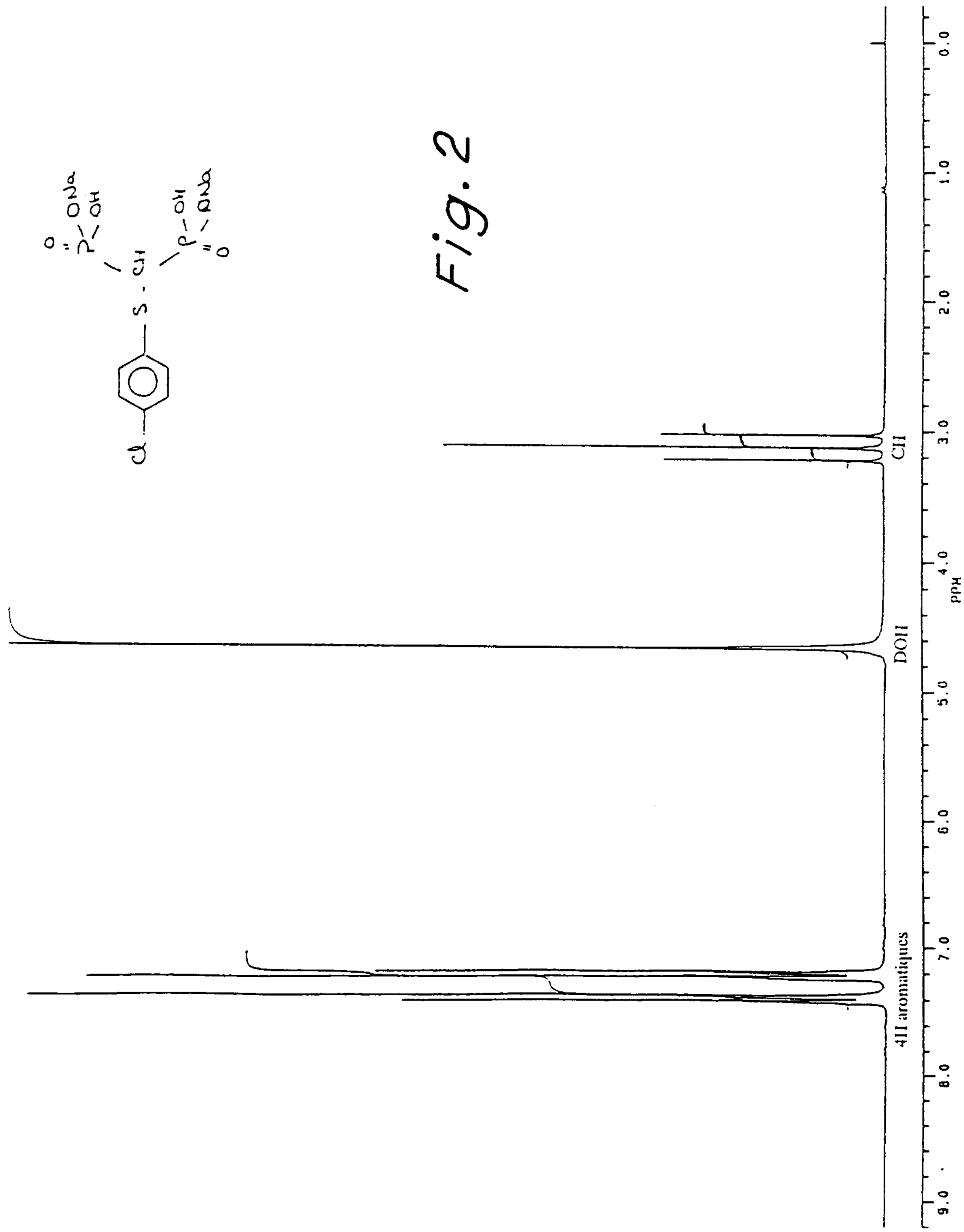


Fig. 2

Fig. 3

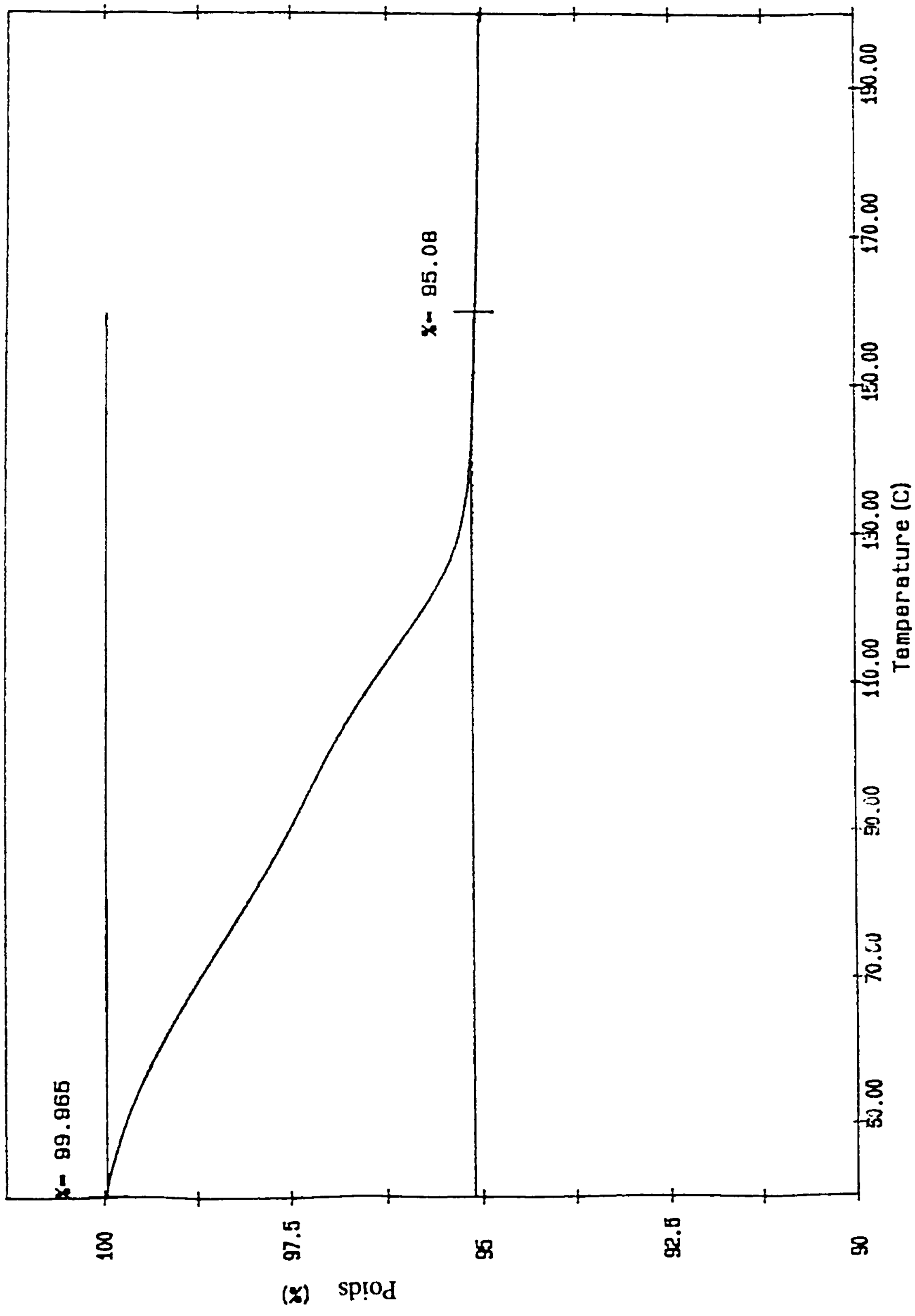
%= 99.965
%= 95.08
100
97.5
95
92.5
90
Poids (%)
50.00
70.00
90.00
110.00
130.00
150.00
170.00
190.00
Temperature (C)

Fig. 4

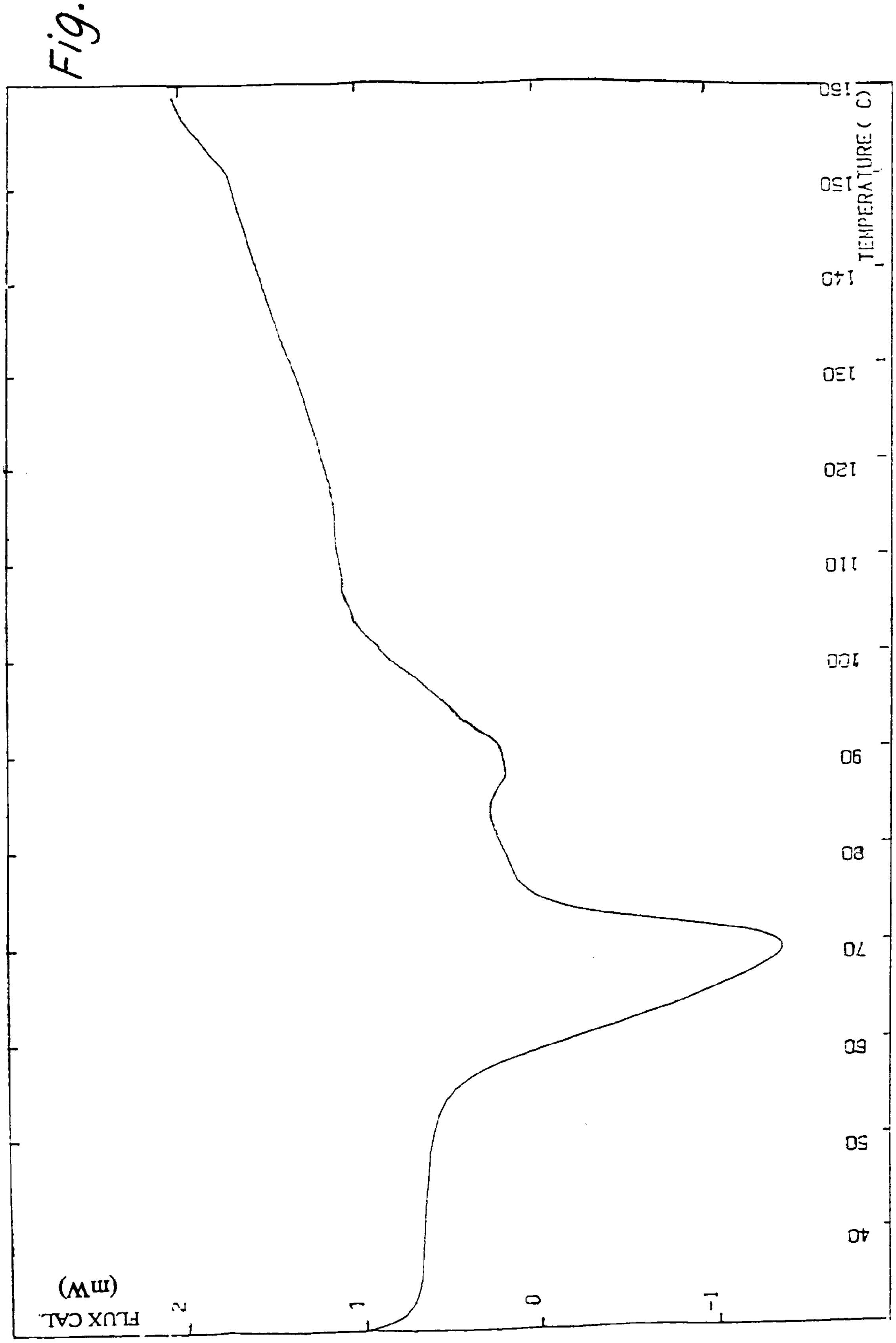
FLUX CAL.
(mW)
2
1
0
-1
TEMPERATURE (°C)
40
50
60
70
80
90
100
110
120
130
140
150
160

Fig. 5

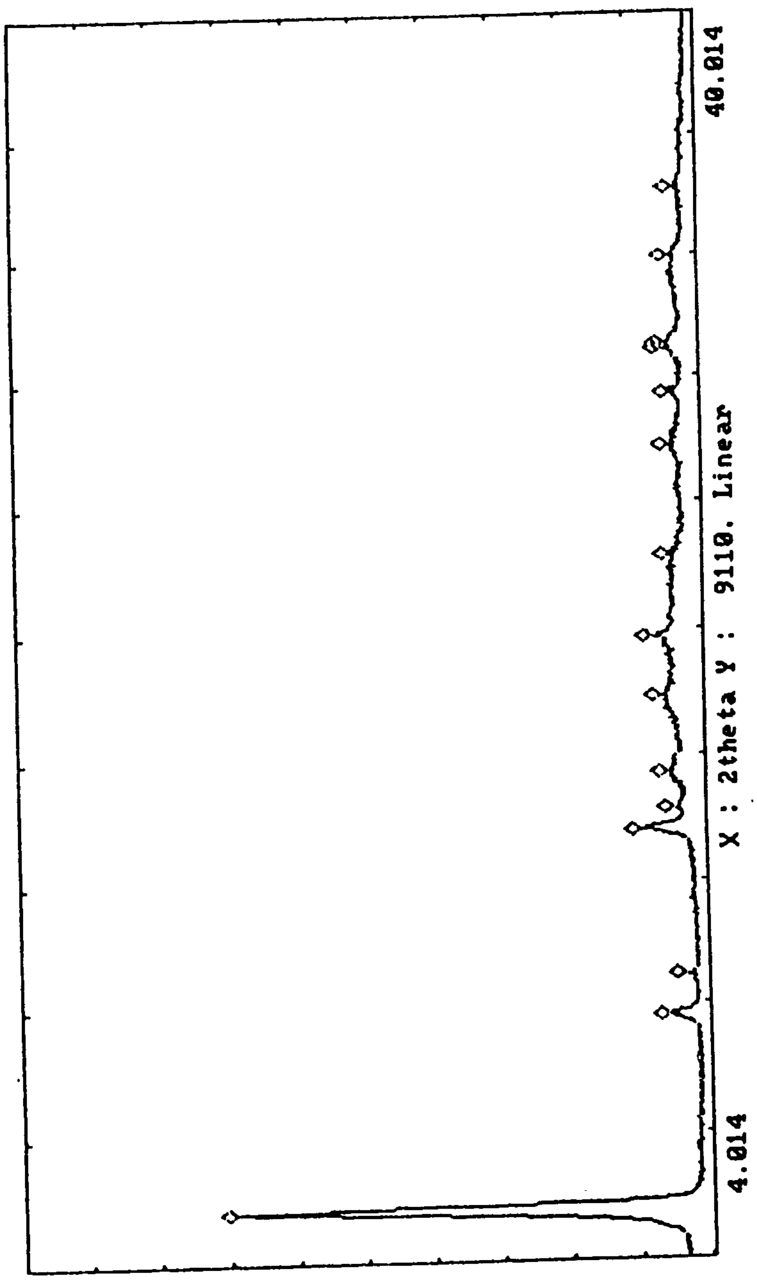

Fig.6

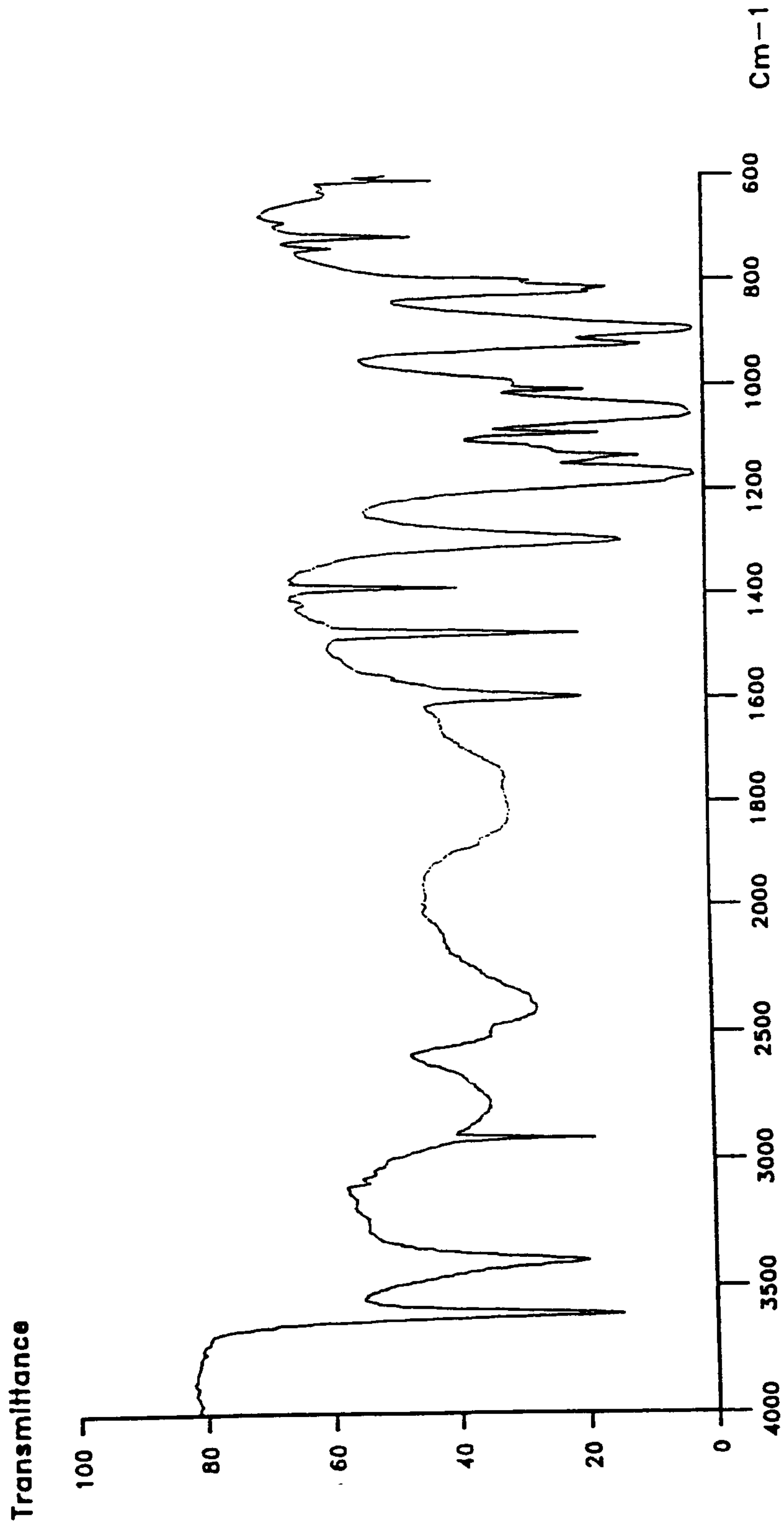
Transmittance
100
80
60
40
20
0
4000
3500
3000
2500
2000
1800
1600
1400
1200
1000
800
600
Cm-1

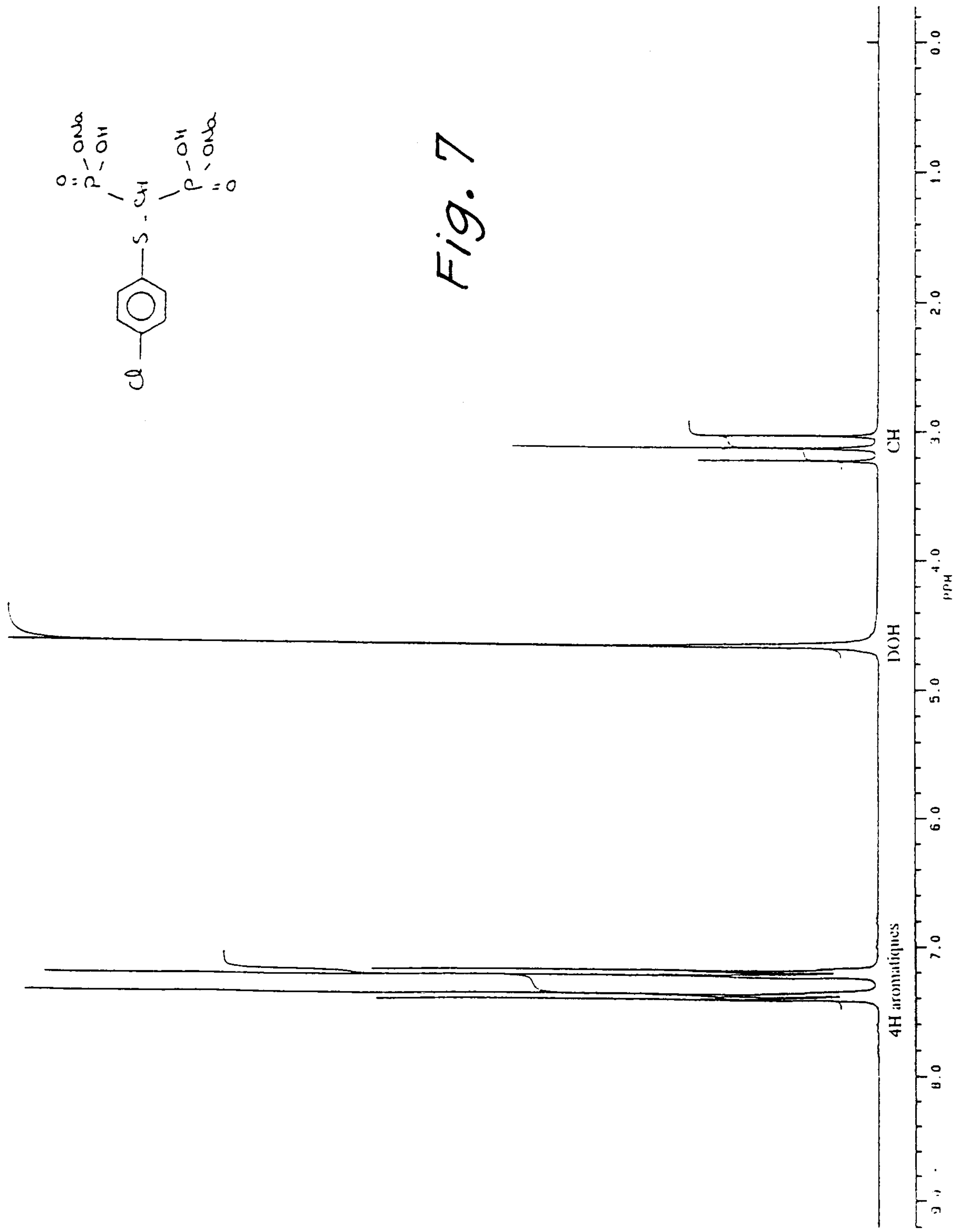


Fig. 7

Fig. 8

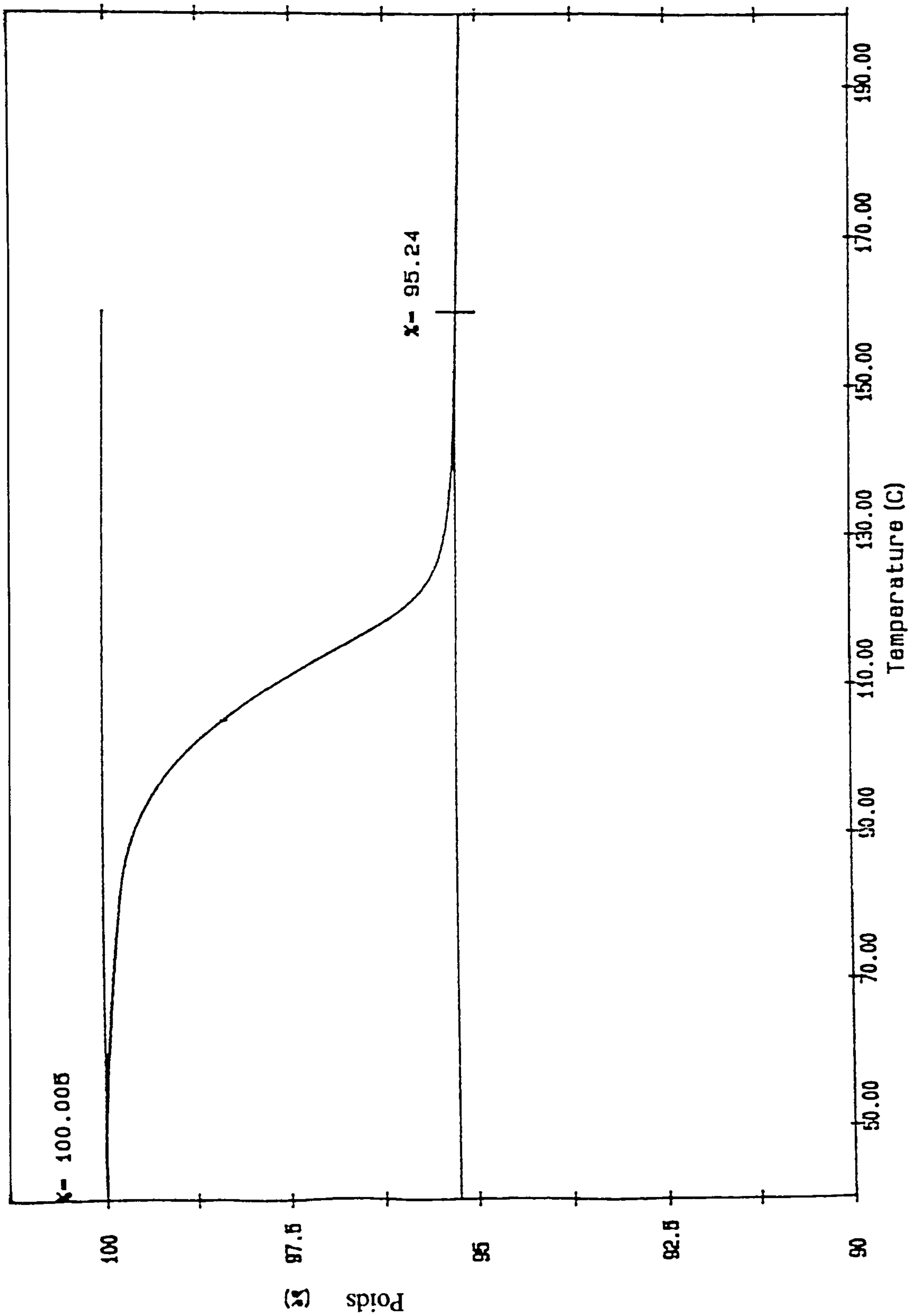
x- 100.005
x- 95.24
100
97.5
95
92.5
90
Poids (%)
50.00
70.00
90.00
110.00
130.00
150.00
170.00
190.00
Temperature (C)

Fig. 9

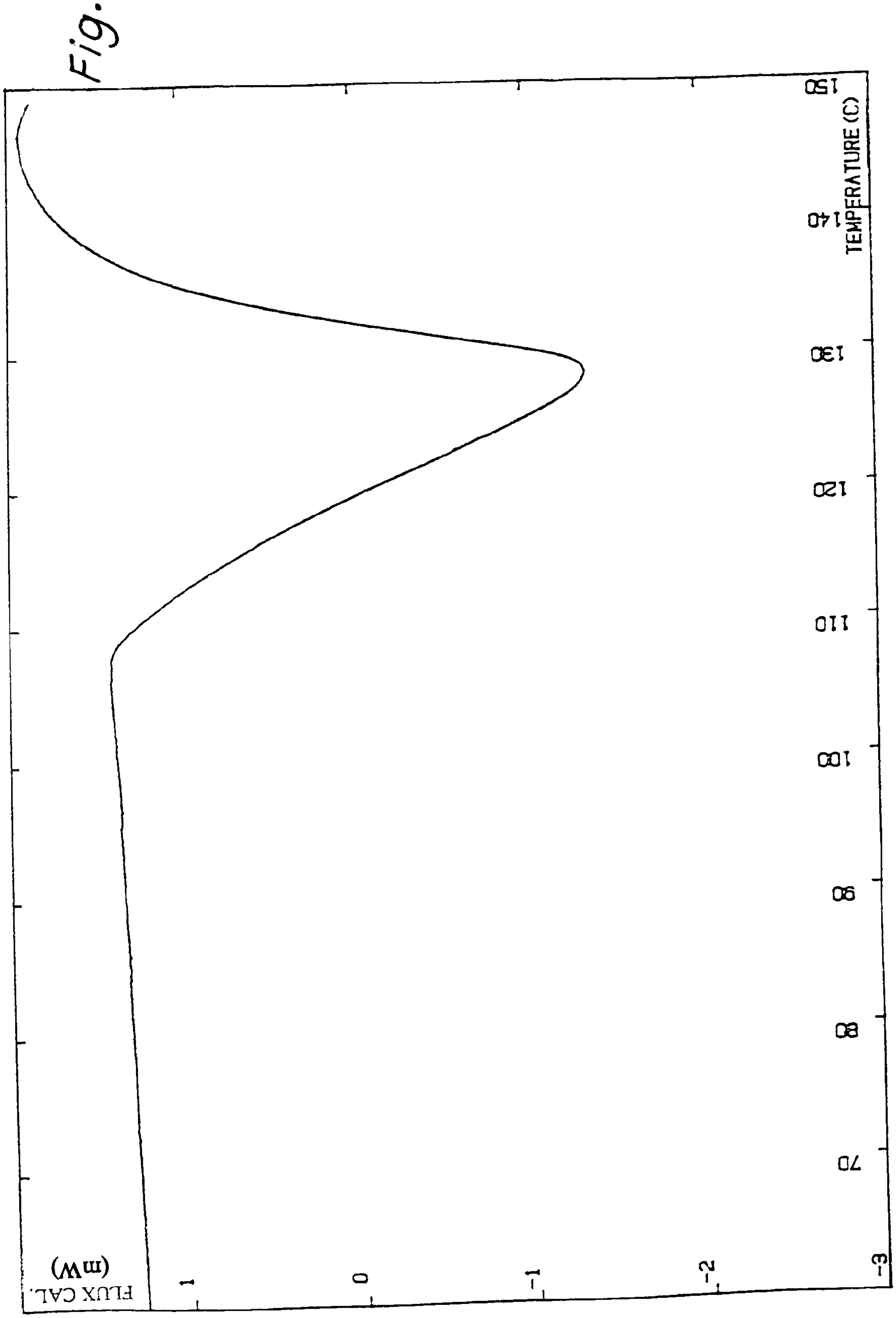
FLUX CAL.
(mW)
1
0
-1
-2
-3
70
80
90
100
110
120
130
140
150
TEMPERATURE (C)

Fig. 10

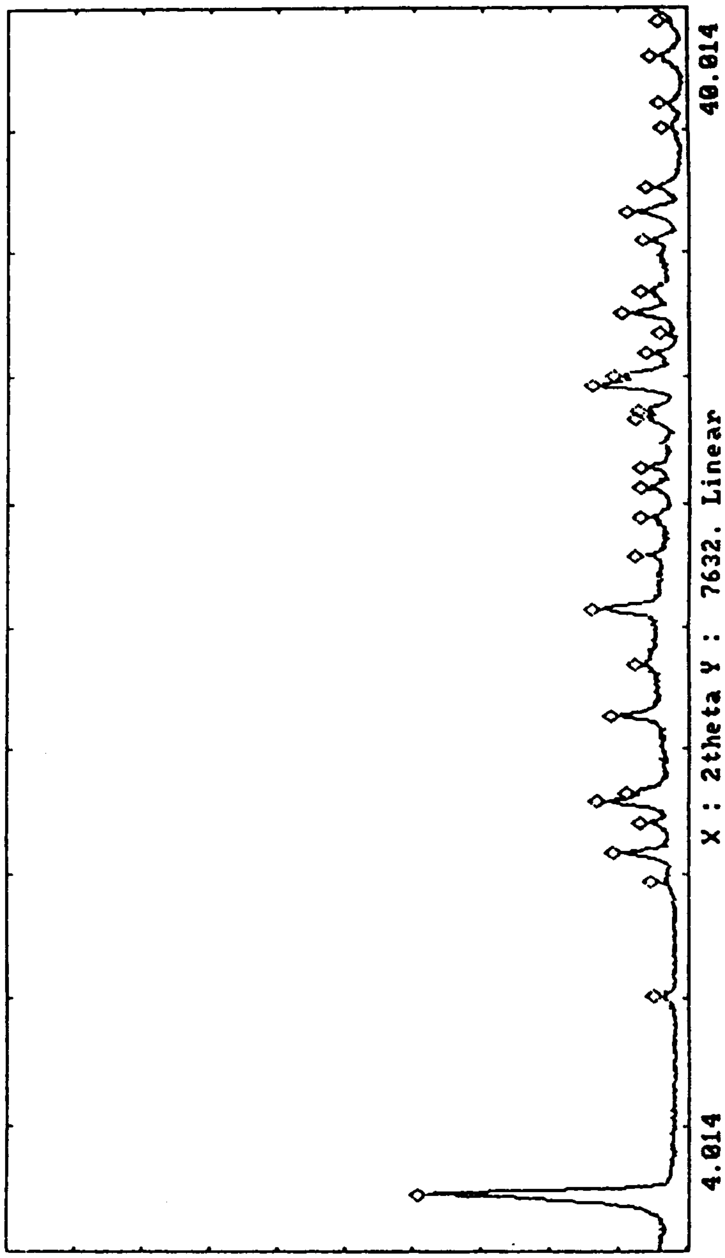

Office européen des brevets

# RAPPORT DE RECHERCHE EUROPEENNE

Numero de la demande
EP 93 40 1995

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.5) |
|---|---|---|---|
| Y | BULLETIN DE LA SOCIETE CHIMIQUE DE FRANCE no. 1 , 1988 , PARIS FR pages 49 - 55 M. BONNERY 'Etude des propriétés acido-basiques et complexantes de l'acide (chloro-4 phenylthio) methylene diphosphonique avec le magnesium et le calcium' * le document en entier * --- | 1-10 | C07F9/38<br>A61K31/66 |
| D,Y | EP-A-0 100 718 (SANOFI S.A.) * le document en entier * --- | 1-10 | |
| A | US-A-4 922 007 (GERARD R. KIECZYKOWSKI) * le document en entier * ----- | 1,10 | |

DOMAINES TECHNIQUES RECHERCHES (Int.Cl.5)
C07F
A61K

Le présent rapport a été établi pour toutes les revendications

| Lieu de la recherche | Date d'achèvement de la recherche | Examinateur |
|---|---|---|
| LA HAYE | 4 Novembre 1993 | BESLIER, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)